# EUROPEAN PATENT SPECIFICATION

(11) **EP 1 506 778 B1**
(45) Date of publication and mention of the grant of the patent: **26.03.2014**
(21) Application number: 03752675.3
(22) Date of filing: 16.05.2003
(51) Int. Cl.: A61K 31/202, A61P 1/16, A61P 35/00, A61P 43/00

(54) **TGF-a EXPRESSION INHIBITORS**
TGF-a EXPRESSIONSHEMMER
INHIBITEURS DE L'EXPRESSION DU TGF-a

(30) Priority: 17.05.2002 JP 2002142862
(43) Date of publication of application: 16.02.2005
(73) Proprietor: Kowa Company, Ltd., Nagoya-shi, Aichi 460-8625 (JP)
(72) Inventor: KAGAWA, Masataka, NIKKEN CHEMICALS CO., LTD., Saitama-shi, Saitam (JP); SANO, Tetsuro, NIKKEN CHEMICALS CO., LTD., Saitama-shi, Saitam (JP); ISHIBASHI, Naoto, NIKKEN CHEMICALS CO., LTD., Saitama-shi, Saitam (JP)
(74) Representative: Godemeyer Blum Lenze - werkpatent
(86) International application number: PCT/JP2003/006116
(87) International publication number: WO 2003/097034

(56) References cited:
- MUTO Y ET AL: "Antitumor activity of a novel synthetic compound polyprenoic-acid derivative" DERMATOLOGICA, S.KARGER, BASEL, CH, vol. 169, no. 4, 1 January 1984 (1984-01-01), page 241, XP008113320 ISSN: 0011-9075
- MUTO Y: 'CHEMOPREVENTION OF HEPATOMA WITH POLYPRENOIC ACIDS' UICC (UNION INTERNATIONALE CONTRE LE CANCER, INTERNATIONAL UNION AGAINST CANCER). 14TH INTERNATIONAL CANCER CONGRESS, vol. 3, 1986, page 847, XP008113324
- NAKAMURA N.: 'Apoptosis in human hepatoma cell line induced by 4,5-didehydro geranylgeranoic acid (acyclic retinoid) via down-regulation of transforming growth factor-alpha' BIOCHEMICAL AND BIOPHYSICAL RESEARCH COMMUNICATIONS vol. 219, no. 1, 1996, pages 100 - 104, XP002969927
- MUTO Y.: 'Prevention of second primary tumors by an acyclic retinoid, polyprenoic acid, in patients with hepatocellular carcinoma' NEW ENGLAND JOURNAL OF MEDICINE vol. 334, no. 24, 1996, pages 1561 - 1567, XP000884177
- OKUNO M.: 'Retinoids exacerbate rat liver fibrosis by inducing the activation of latent TGF-<SYM98> in liver stellate cells' HEPATOLOGY vol. 26, no. 4, 1997, PHILADELPHIA, pages 913 - 921, XP002969928
- DI BISCEGLIE A.M.: 'Hepatocellular carcinoma' HEPATOLOGY vol. 28, no. 4, 1998, pages 1161 - 1165, XP002969929
- ISHIWARI K.: 'Effects of a synthetic retinoid, polyprenoic acid (E5166), on phenotypic expression of cultured mesangial cells' KYOTO-FURITSU IKA DAIGAKU ZASSHI vol. 106, no. 3, 1997, pages 273 - 283, XP002969930
- Yun-Chi Yeh ET AL: "Elevation of transforming growth factor alpha and its relationship to the epidermal growth factor and alpha-fetoprotein levels in patients with hepatocellular carcinoma", Cancer Res, 1 January 1987 (1987-01-01), pages 896-901, XP055079830, Retrieved from the Internet: URL:http://cancerres.aacrjournals.org/cont ent/47/3/896.full.pdf [retrieved on 2013-09-18]

## Description

### Technical Field

The present invention relates to an inhibitor against expression of transforming growth factor-α (hereinafter sometimes referred to as "TGF-α" in the specification) which comprises as an active ingredient a polyprenyl compound. The present invention also relates to an inhibitor against transformation of a hepatitis cell or a cirrhosis cell and an inhibitor against onset, recurrence (second oncogenesis), and malignant alteration of hepatoma on the basis of the aforementioned inhibition of TGF-α expression.

### Background Art

Malignant neoplasm mortality in 1999 is as high as 296 thousands, which is the worst cause of death in Japan. Among them, more than 30 thousands died from hepatocellular carcinoma. The number has been increasing year by year, and has increased threefold for the recent 20 years. As main causes of hepatocellular carcinoma appeared in Japan, 90% or more of the disease is considered to be caused by continuous infection (chronic hepatitis) with hepatitis B virus (hereinafter sometimes referred to as "HBV" in the specification) or with hepatitis C virus (hereinafter sometimes referred to as "HCV" in the specification). As for hepatoma, recurrence (second oncogenesis) at a yearly ratio of about 20 to 25% after therapeutic treatment is observed, and thus this disease results in a poor prognosis. Accordingly, important future objects include suppression of hepatic oncogenesis from chronic hepatitis and recurrence of hepatoma after treatment, as well as earlier detection and treatment of hepatoma.

Transforming growth factor-α (TGF-α) is believed to be significantly involved in hepatic oncogenesis and malignant alteration (promotion/progression) of hepatoma cells. It is reported that TGF- a expression level in the liver is associated with severity of inflammation in virus hepatitis, and TGF-α overexpression in cirrhosis caused by hepatitis viruses is related to replication of hepatitis viruses. In a hepatic tissue of a patient suffering from a chronic hepatic disease caused by HBV or HCV infection, a higher TGF-α expression is observed as compared to a healthy person. A high expression of TGF-α is also observed in a cancer tissue of a hepatoma patient and non-tumor tissues surrounding the cancer. Therefore, a possibility is suggested that TGF-α has important roles in the process from hepatitis virus infection to onset and development of hepatoma.

It is experimentally observed that transformations of rat liver cells are associated with promotions of TGF-α expression, and that TGF-α is expressed in a high level in hepatic cells which are transformed with a hepatic chemical carcinogen and a hepatitis virus. Further, in a transgenic mouse with overexpressed TGF-α, a high ratio of spontaneous onset of hepatoma is observed, as well as a remarkable acceleration of onset of hepatic oncogenesis by a chemical carcinogen and a cancer gene is observed in this mouse.

As described above, TGF-α is significantly involved in hepatic oncogenesis and malignant alteration of hepatoma cells. Therefore, a proposal has been made that a new strategy of treatment of hepatoma can be developed by setting TGF-α as a target.

Although a precise mechanism of hepatoma onset in a patient suffering from chronic virus hepatitis has not yet been elucidated, a possibility of important roles of a hepatic stem cell (oval cell: hereinafter sometimes referred to as "oval cell" in the specification) is suggested. Proliferations of oval cells are observed in patients suffering from chronic hepatitis or cirrhosis, and an increase of number of oval cells in proportion to severity of hepatic pathological condition is observed. These phenomena are induced on the basis of the increase of activity of oval cell division and proliferation due to chronic inflammation in the liver caused by hepatitis viruses. At the same time, hepatitis viruses act as an oncogenic initiator on oval cells. Accordingly, a mechanism is suggested that an oval cell initiated by a hepatitis virus promotes its activity of division and proliferation (promotion) by chronic inflammation, which leads to canceration (oncogenesis).

Further, in many experiments of hepatic chemical oncogenesis by using animals, some reports indicate that hepatoma is derived from dedifferentiated hepatic cells, whilst other reports indicate a possibility that hepatic oncogenesis is derived from oval cells as an origin, because a phenotype of a hepatoma cell has resemblance to that of an oval cell.

A lot of studies are reported on relations between oval cells and TGF-α. TGF-α is a substance promoting division of oval cell (mitogen). TGF-α produced in oval cells, per se, or in surrounding hepatic tissues stimulates proliferation of oval cells via epidermal growth factor receptor (EGFR) which is a receptor of TGF-α. In addition, an oval cell, which is remarkably observed in virus chronic hepatitis of a human, is revealed to be a target cell of hepatitis virus infection and at the same time accompanied with TGF-α hyperexpression. Therefore, a possibility is suggested that oval cells, per se, may change to hepatoma cells through the action of hepatitis viruses at an early stage of oncogenesis (initiation) and the involvement of TGF-α as an promoter after the initiation.

As described above, the interaction between the presence of a virus in an oval cell and TGF-α expression is an important factor for inducing the canceration of the oval cell. In addition, a probability is suggested that TGF-α overexpressed in hepatitis/cirrhosis tissues surrounding oval cells promotes the canceration of oval cells via the receptors. Therefore, suppression of TGF-α expression in hepatitis/cirrhosis tissues, as well as reduction of TGF-α expression in oval cells observed in virus chronic hepatitis, is considered to be a extremely important mechanism which deactivates the oval cell as being a precursor cell of hepatoma and leads to the control of a hepatic oncogenesis.

As explained above, TGF-α is closely associated with transformation of cells of hepatitis and cirrhosis, and with oncogenesis in a liver and malignant alteration of hepatoma cells. Accordingly, it is highly probable that a control of TGF-α overexpressed in hepatic tissue cells and oval cells in the liver may lead to suppression of transformation of cells of hepatitis and cirrhosis, and to suppression of oncogenesis in the liver and malignant alteration to hepatoma cells.

(2E,4E,6E,10E)-3,7,11,15-tetramethyl-2,4,6,10,14-hexadecapentaenoic acid (development code "NIK-333"), a class of polyprenyl compound, is known to have affinities for retinoic acid-binding proteins and retinoic acid receptors, as well as an action for inducing differentiation and apoptosis in hepatoma cells. Clinically, it has been reported that NIK-333 significantly suppressed recurrence after radical cure of hepatoma by a long-term administration for one year, which reveals that the substance has an inhibitory action against recurrence of hepatoma. Further, during the above administration, almost no side effect is observed such as dysfunction of liver and those with other retinoids, thus NIK-333 is a safe medicament (N. Eng. J. Med. 334, 1561-1567 (1996)).

The document Muto Y. et al. "Antitumor activity of a novel synthetic compound polyprenoic-acid derivative", DERMATOLOGICA, Vol. 169 No. 4, page 241 (1984)) describes the antitumor activity of a novel synthetic compound 3,7,11,15-tetramethyl-2,4,6,10,14-hexadecapentaenoic acid which is a polyprenoic acid derivative and named E-5166 in the reference. The reference describes that the compound has an in-vivo inhibitory effect on the development of skin papillomas in mice and hepatocellular carcinomas induced by γ-methyl DAB in rats as well as in spontaneous hepatoma of mice. The authors conclude that a local deficiency of the vitamin A plays a role in the early development of the cancer.

The document does not describe that the polyprenyl compound can be used as an inhibitor against TGF-α expression in a hepatic tissue cell transformed by a hepatic chemical carcinogen.

The document Muto Y. ("Chemoprevention of heptoma with polyprenoic acids" Symposia and free communications, Volumes 1, 2, 3, page 847 (1986)) describes the chemoprevention of hepatoma with polyprenoic acid. It is disclosed in the reference that oral administration of TNCP (tetramethylmethoxyphenyl analogs of retinoic acid) and polyprenoic acid significantly reduced tumor incidences of experimental hepatomas induced by chemical carcinogen in rats as well as in spontaneous hepatoma-bearing mice genetically determined.

The reference does not describe that the polyprenyl compound can be used as an inhibitor against TGF-α expression in hepatic tissue cell transformed by hepatic chemical carcinogen.

It is already reported that a polyprenyl compound inhibits TGF- a expression in an established hepatoma cell (Biochem. Biophys. Res.Commun. 219, 100-104 (1996)). However, it is totally unknown that, in a hepatic tissue in vivo, a polyprenyl compound inhibits TGF-α whose expression is promoted in the hepatic tissue by the administration of a hepatic chemical carcinogen, and that the compound suppresses TGF-α expression in an oval cell which is considered as a precursor cell of hepatoma.

### Disclosure of the Invention

An object of the present invention is thus to provide an inhibitor against transformation of hepatitis and/or cirrhosis cells by inhibiting TGF- α expression, and an inhibitor against oncogenesis in a liver and an inhibitor against malignant alteration of carcinoma.

The inventors of the present invention have conducted various researches to find an inhibitor against transformation of hepatitis and/or cirrhosis cells (an inhibitor against malignant alteration of hepatitis or cirrhosis), an inhibitor against oncogenesis and an inhibitor against malignant alteration of carcinoma in the liver. As a result, they found that a polyprenyl compound inhibits TGF-α expression in hepatic tissue cells transformed by a hepatic chemical carcinogen and TGF-α expression in an oval cell which is strongly suggested as a possible precursor cell of a hepatoma.

The present invention thus provides:
(1) A polyprenyl carboxylic acid as an active ingredient for use in the inhibition of onset, recurrence (second oncogenesis), and malignant alteration of hepatoma by inhibiting TGF- α expression in a hepatic tissue cell transformed by a hepatic chemical carcinogen, wherein the polyprenyl carboxylic acid is 3,7,11,15-tetramethyl-2,4,6,10,14-hexadecapentaenoic acid.
(2) A polyprenyl compound according to (1), wherein the polyprenyl carboxylic acid is (2E,4E,6E,10E)-3,7,11,15-tetramethyl-2,4,6,10,14-hexadecapentaenoic acid.
(3) A polyprenyl compound according to (1) or (2), wherein the hepatic tissue cell is an oval cell.
(4) A polyprenyl compound according to any one of (1) to (3), which is used for inhibiting transformation of a hepatitis and/or cirrhosis cell.
(5) A polyprenyl compound according to any one of (1) to (4), which is in a form of a pharmaceutical composition comprising a pharmaceutically acceptable carrier.
(6) A polyprenyl compound according to (5), which is a pharmaceutical composition for oral administration.

The polyprenyl carboxylic acid according to the present invention, i.e. 3,7,11,15-tetramethyl-2,4,6,10,14-hexadecapentaenoic acid is for administration to a mammal including human.

### Brief Explanation of Drawings

Fig. 1 shows a photograph of a hepatic tissue of a non-treated animal. No expression of TGF-α -positive oval cells is observed.
Fig. 2 is a photograph of oval cells expressed in a hepatic tissue of a control group (administered only with 3'-MeDAB as a hepatic chemical carcinogen) which give a strongly positive TGF-α observation in the cytoplasm (the areas stained with deep brown color indicate TGF-α expressions).
Fig. 3 is a photograph showing that oval cells expressed with 3'-MeDAB give weakly positive TGF-α observation by the administration of 80 mg/kg/day of NIK-333 (the areas stained with deep brown color are smaller than those in Fig. 2), indicating a reduced TGF-α expression.
Fig. 4 shows a photograph of a hepatic tissue of a non-treated animal. Almost no expression of TGF-α is observed.
Fig. 5 is a photograph showing TGF-α expression in a non-tumor hepatic tissue of a control group (administered only with DEN as a hepatic chemical carcinogen). The hepatic tissues surrounding the central vein give strongly positive TGF- a observation (the areas stained with deep brown color indicate TGF-α expressions).
Fig. 6 is a photograph showing that TGF-α expressed with DEN in hepatic tissues surrounding the central vein is found to be almost negative by the administration of 80 mg/kg/day of NIK-333 (almost no area stained with deep brown color is observed).
Fig. 7 shows a photograph of a hepatic tissue of a non-treated animal. Almost no TGF- a expression is observed.
Fig. 8 is a photograph showing TGF- a expression by DEN administration in non-tumor hepatic tissues of a control group. The hepatic tissues surrounding the central vein give strongly positive TGF- a observations (the areas stained with deep brown color indicate TGF-α expressions).
Fig. 9 is a photograph showing that TGF-α expressed with DEN in hepatic tissues surrounding the central vein is found to be almost negative by the administration of 80 mg/kg/day of NIK-333 (almost no area stained with deep brown color is observed).
Fig. 10 shows a photograph of a hepatic tissue of a non-treated animal. Expression of TGF-α-positive oval cells is not observed.
Fig. 11 is a photograph showing that oval cells expressed in a hepatic tissue of a control group by 3'-MeDAB administration give strongly positive TGF-α observations in the cytoplasm (the areas stained with deep brown color indicate TGF-α expressions).
Fig. 12 is a photograph showing that the number of oval cells with strongly positive TGF-α observation, expressed with 3'-MeDAB, is reduced by the administration of 80 mg/kg/day of NIK-333 (the number of cells stained with deep brown color is less than that in Fig. 11).
Fig. 13 shows a photograph of a hepatic tissue of a non-treated animal. Cells which give apparent positive TGF-α observations are not observed.
Fig. 14 is a photograph showing that oval cells expressed by the administration of D-galactosamine hydrochloride in a hepatic tissue of a control group give strongly positive TGF-α observation in the cytoplasm (the areas stained with deep brown color indicate TGF-α expressions).
Fig. 15 is a photograph showing that oval cells expressed with D-galactosamine hydrochloride give weakly positive TGF- a observation by the administration of 200 mg/kg/day of NIK-333 (the areas stained with deep brown color are smaller than those in Fig. 14), which reveals reduction of TGF-α expression.

### Best Mode for Carrying out the Invention

The polyprenyl compound used in the present invention is 3,7,11,15-tetramethyl-2,4,6,10,14-hexadecapentaenoic acid. An example of the most preferable compound includes (2E,4E,6E,10E)-3,7,11,15-tetramethyl-2,4,6,10,14-hexadecapentaenoic acid (NIK-333).

A polyprenyl compound used in the present invention can be prepared by a known method (Japanese Patent Publication (Kohyo) No. 63-32058 (1988), J.Chem.Soc. (C), 2154, 1966).

As the inhibitor against TGF-α expression of the present invention, a pharmaceutical composition comprising a polyprenyl compound for use according to the present invention is generally prepared, and administered orally or parenterally whichever is suitable. Examples of formulations of the pharmaceutical composition suitable for oral administration include tablets, granules, capsules, soft capsules, pills, powders, and liquids. Examples of formulations of the pharmaceutical composition suitable for parenteral administration include injections and suppositories. These pharmaceutical compositions can be prepared by using a polyprenyl compound as defined in claim 1 or a pharmacologically acceptable salt thereof and one or more pharmaceutical carriers according to an ordinary method.

For example, for a medicament suitable for oral administration, a desired pharmaceutical composition can be prepared by using, as a pharmaceutical carrier, excipients such as lactose, glucose, corn starch, sucrose; disintegrants such as calcium carboxymethylcellulose and hydroxypropylcellulose; lubricants such as calcium stearate, magnesium stearate, talc, polyethylene glycol, and hydrogenated oil; binders such as hydroxypropylcellulose, hydroxypropylmethylcellulose, carboxymethylcellulose, polyvinylalcohol, gelatin, Arabic gum; wetting agents such as glycerol and ethyleneglycol; and further, surfactants and flavoring agents, if necessary.

For a medicament suitable for parenteral administration, diluents such as water, ethanol, glycerol, propylene glycol, polyethylene glycol, vegetable oil, agar, and gum tragacanth can be used as a pharmaceutical carrier. Solubilizing agents, suspending agents, emulsifying agents, stabilizing agents, buffering agents, isotonizing agents, preservatives, soothing agents or the like can be used, if necessary.

A dose of the inhibitor against TGF-a expression of the present invention is not particularly limited. For example, the dose for oral administration may be 50 -1200 mg, preferably 300-900 mg per day for an adult, and the dose for parenteral administration may be 1 -1200 mg, preferably 5-900 mg per day for an adult. Desired inhibitory actions can be expected by the administration of each of the above doses once or 2 to 3 times as divided portions.

### Examples

The present invention will be explained in more detail with reference to examples. In the following examples, (2E,4E,6E,10E)-3,7,11,15-tetramethyl-2,4,6,10,14-hexadecapentaenoic acid (hereinafter referred to as "NIK-333") was used as a polyprenyl compound.

### Example 1

### Effects on induction of TGF- a positive oval cells of a rat by treatment with 3'-methyl-4-dimethylaminoazobenzene (3'-MeDAB) as a hepatic chemical carcinogen

Male Fischer rats (F344/N Slc, 6-week old) were used as an animal, and the rats were given with solid feed prepared to contain a hepatic chemical carcinogen 3'-MeDAB in a ratio of 0.06% for four weeks to induce oval cells. NIK-333 was suspended in soybean oil, and a dose of 80 mg/kg was orally administered to each rat for four weeks (once/a day) along with the administration of 3'-MeDAB. To a control group, soybean oil (5 mL/kg) was orally administered. After 4 weeks from the start of the administration of 3'-MeDAB, livers were extirpated under anesthesia. The livers were subjected to 10 % formalin fixation and paraffin embedding, and further to immunological staining by using an anti TGF- a antibody. TGF- a expression levels were analyzed under microscope.

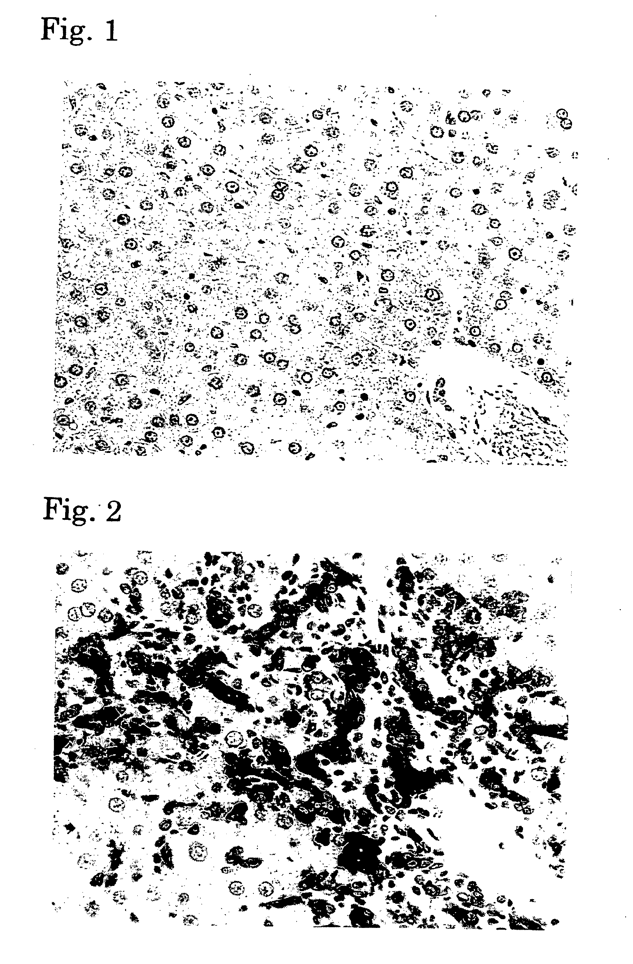

Figs. 1 to 3 are photographs showing inhibitory effects on induction of TGF- a positive oval cells induced by the four-week administration of 3'-MeDAB, when 80 mg/kg/day of NIK-333 was simultaneously administered for four weeks (immunohistological staining by using the anti TGF- a antibody). Similar observations were obtained in other animals which were analyzed at the same time.

Fig. 1 shows a photograph of a hepatic tissue of a non-treated animal. No expression of oval cells showing TGF-α-positive is observed.

Fig. 2 shows a strongly positive TGF-α observation in the cytoplasm of oval cells expressed in a hepatic tissue of the control group (administered only with 3'-MeDAB).

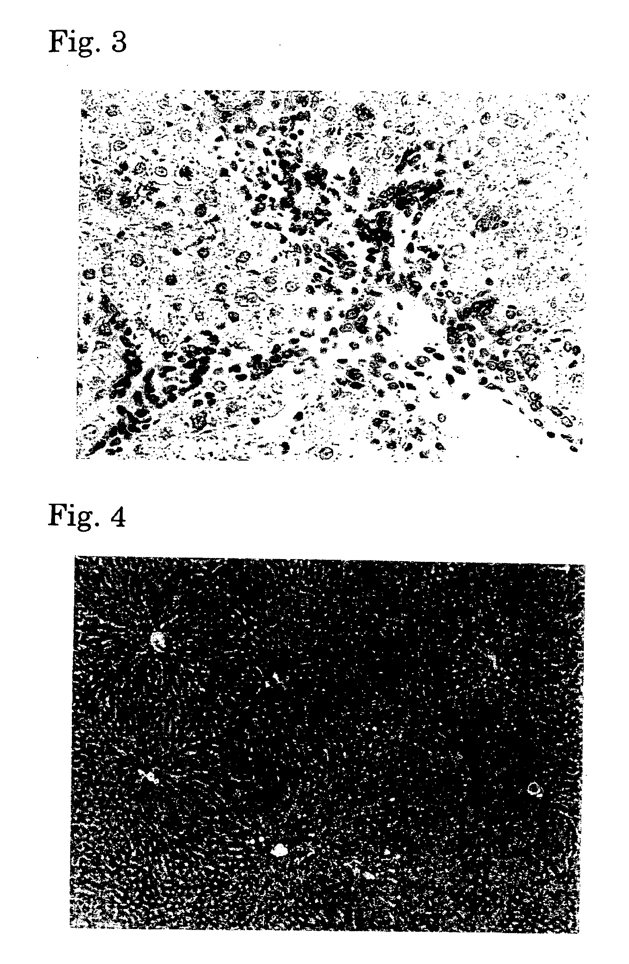

Fig. 3 shows that oval cells expressed with 3'-MeDAB gave weakly positive TGF-α observation by the administration of 80 mg/kg/day of NIK-333, indicating a reduced TGF-α expression.

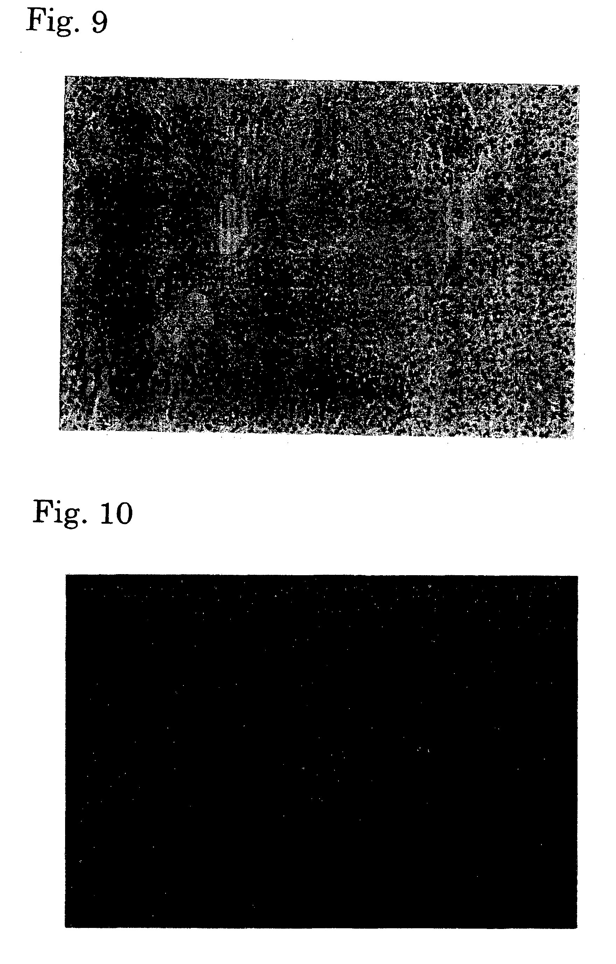

In the same experiment, other portions were photographed, and the results were obtained as shown in Figs. 10 to 12.

Fig. 10 shows a hepatic tissue of a non-treated animal. Expression of oval cells showing TGF-α-positive is not observed.

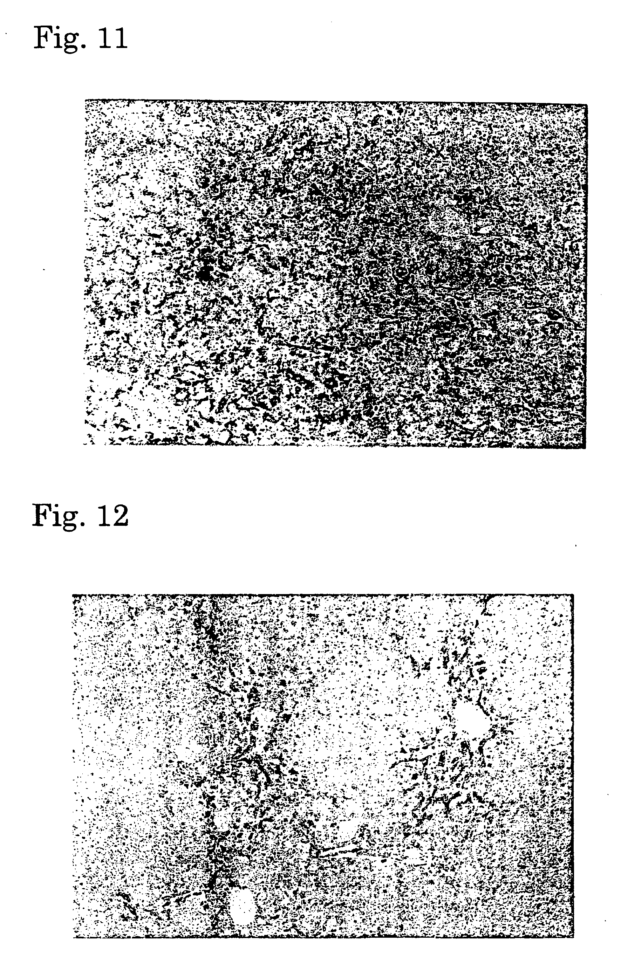

Fig. 11 shows a strongly positive TGF- a observation in the cytoplasm of oval cells expressed by 3'-MeDAB administration in a hepatic tissue of the control group.

Fig. 12 shows that the number of oval cells with strongly positive TGF- a observation, expressed with 3'-MeDAB, was reduced by the administration of 80 mg/kg/day of NIK-333.

### Example 2

### Effects on TGF-α expression in a non-tumor hepatic tissue of a rat after administration of N-Nitrosodiethylamine (DEN) as a hepatic chemical carcinogen

Male Fischer rats (F344/N Slc, 6-week old) were used as an animal, and the rats were administered with a hepatic chemical carcinogen DEN for 5 weeks in a form of drinking water (concentration: 40 ppm), and then bred with normal drinking water for successive 15 weeks to induce hepatocellular carcinoma. NIK-333 was suspended in soybean oil, and a dose of 80 mg/kg was orally administered to each rat for 14 weeks (once/a day) from one week after the end of the administration of DEN. To a control group, soybean oil (5 mL/kg) was orally administered. After 20 weeks from the start of the administration of DEN, livers were extirpated under anesthesia. The livers were subjected to 10 % formalin fixation and paraffin embedding, and further to immunological staining by using an anti TGF-α antibody. TGF-α expression levels were analyzed under microscope.

Figs. 4 to 6 are photographs showing inhibitory effects on TGF-α expression in a non-tumor hepatic tissue, when the administration was ceased for one week after the 5-week treatment with DEN, and then 80 mg/kg/day of NIK-333 was administered for 14 weeks (immunohistological staining by using an anti TGF-α antibody). Similar observations were obtained in other animals which were analyzed at the same time.

Fig. 4 shows a hepatic tissue of a non-treated animal. Almost no expression of TGF-α was observed.

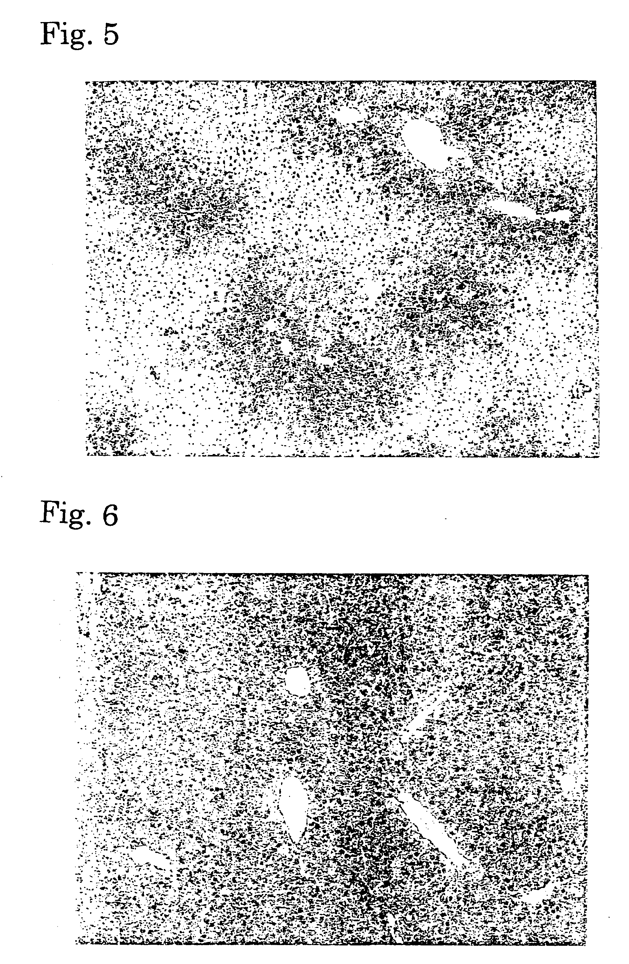

Fig. 5 shows TGF-α expression in a non-tumor hepatic tissue of the control group (administered only with DEN). Strongly positive TGF-α expression was observed in the hepatic tissue surrounding the central vein.

Fig. 6 shows results of almost negative observations of TGF-α after the administration of 80 mg/kg/day of NIK-333, which TGF-α was expressed by DEN in hepatic tissues surrounding the central vein.

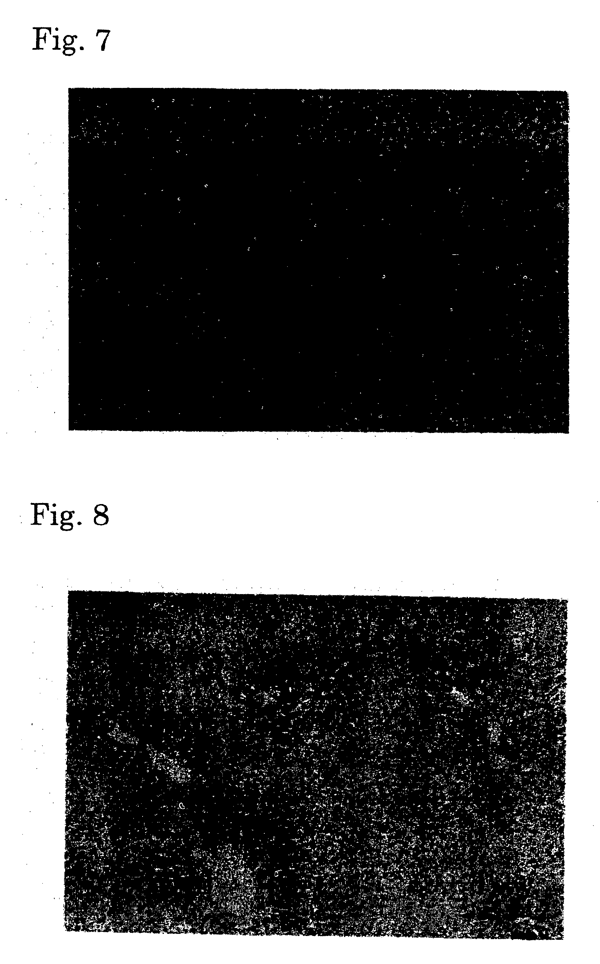

In the same experiment, other portions were photographed, and the results were obtained as shown in Figs. 7 to 9.

Fig. 7 shows a hepatic tissue of a non-treated animal. Almost no expression of TGF-α was observed.

Fig. 8 shows TGF-α expression by DEN administration in non-tumor hepatic tissues of the control group. The hepatic tissue surrounding the central vein gave a strongly positive TGF-α observation.

Fig. 9 shows results of almost negative observations of TGF-α after the administration of 80 mg/kg/day of NIK-333, which TGF-α was expressed by DEN in hepatic tissues surrounding the central vein.

### Example 3

### Effects on TGF-α expression in oval cells in a rat induced by D-galactosamine hydrochloride

Male SD rats (Crj/CD (SD), 6-week old) were used as an animal, and D-galactosamine hydrochloride (5 ml/kg) prepared as 200 mg/mL with physiological saline was intraperitoneally administered once to each rat to induce oval cells. NIK-333 was suspended in soybean oil, and a dose of 200 mg/kg was orally administered to each rat for 1 to 6 days. To the control group, soybean oil (2 mL/kg) was orally administered. From the next day to 7 days after the start of the administration, the livers were extirpated under anesthesia with passage of time. The livers were subjected to 10 % formalin fixation and paraffin embedding, and further to immunological staining by using an anti TGF- a antibody. TGF-α expression levels were analyzed under microscope.

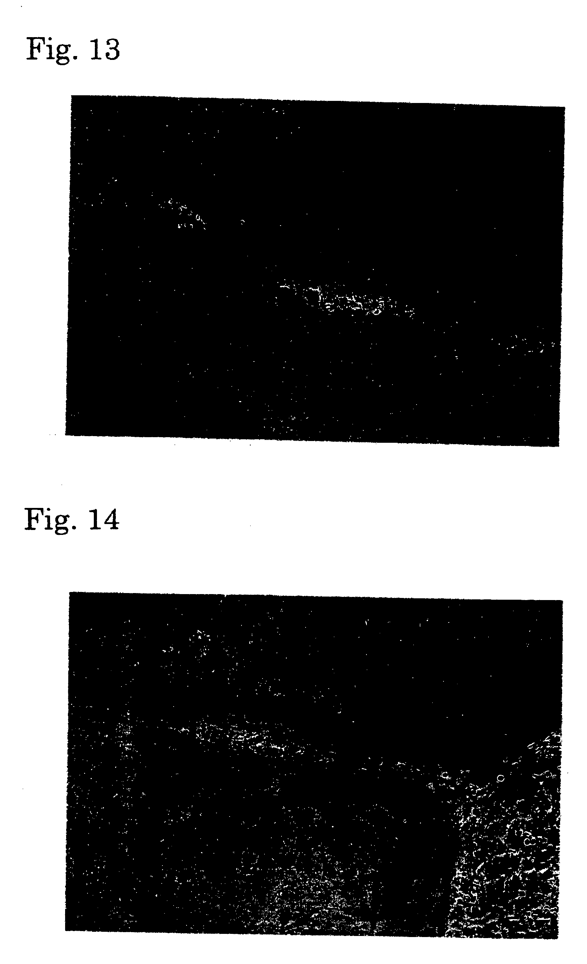

Figs. 13 to 15 are photographs showing inhibitory effects on TGF-α expression in oval cells induced by the administration of D-galactosamine hydrochloride, when 200 mg/kg/day of NIK-333 was administered for four days (immunohistological staining using an anti TGF-α antibody). Similar observations were obtained in other animals which were analyzed at the same time.

Fig. 13 shows a hepatic tissue of a non-treated animal. Cells showing an apparent TGF-α positive observation were not found.

Fig. 14 shows a result of a strongly positive TGF-α observation in the cytoplasm of oval cells expressed by the administration of D-galactosamine hydrochloride in a hepatic tissue of the control group.

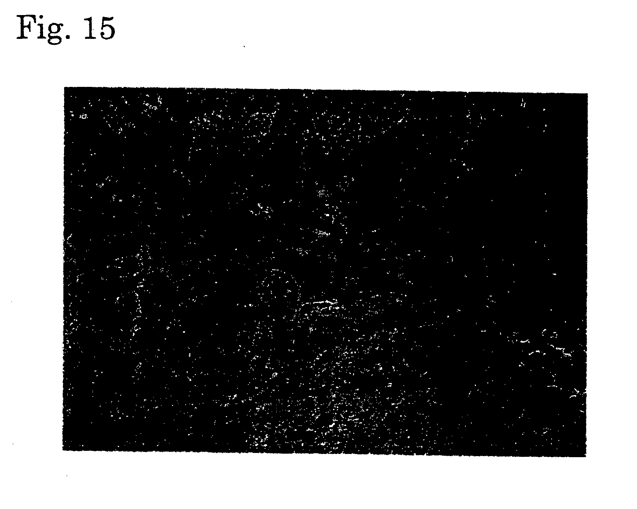

Fig. 15 shows a result of weakly positive TGF- a observation of oval cells expressed by D-galactosamine hydrochloride by the administration of 200 mg/kg/day of NIK-333, indicating reduction of TGF- α expression.

### Industrial Applicability

A polyprenyl compound as defined in clam 1 inhibits transformation of a hepatitis cell or a cirrhosis cell transformed by a hepatic chemical carcinogen, or TGF-α involved in oncogenesis or malignant alteration of tumor cells in a liver. Therefore, a polyprenyl compound as defined in clam 1 can be used as an inhibitor against transformation of a hepatitis cell or a cirrhosis cell transformed by a hepatic chemical carcinogen, as an inhibitor against malignant alteration of hepatitis or cirrhosis, and is useful as an inhibitor against onset, recurrence (second oncogenesis), and malignant alteration of carcinoma in a liver.

## Claims

1. A polyprenyl carboxylic acid as an active ingredient for use in the inhibition of onset, recurrence (second oncogenesis), and malignant alteration of hepatoma by inhibiting TGF-α expression in a hepatic tissue cell transformed by a hepatic chemical carcinogen, wherein the polyprenyl carboxylic acid is 3,7,11,15-tetramethyl-2,4,6,10,14-hexadecapentaenoic acid.

2. A polyprenyl compound for use according to claim 1, wherein the polyprenyl carboxylic acid is (2E,4E,6E,10E)-3,7,11,15-tetramethyl-2,4,6,10,14-hexadecapentaenoic acid.

3. A polyprenyl compound for use according to claim 1 or 2, wherein the hepatic tissue cell is an oval cell.

4. A polyprenyl compound for use according to any one of claims 1 to 3, which is used for inhibiting transformation of a hepatitis and/or cirrhosis cell.

5. A polyprenyl compound for use according to any one of claims 1 to 4, which is in a form of a pharmaceutical composition comprising a pharmaceutically acceptable carrier.

6. A polyprenyl compound for use according to claim 5, which is a pharmaceutical composition for oral administration.

## Patentansprüche

1. Eine Polyprenylcarbonsäure als Wirkstoff zur Verwendung in der Hemmung des Ausbruches, des Wiederauftretens (zweite Onkogenese) und der malignen Änderung von Hepatomen mittels Hemmung der TGF-α-Expression in einer Lebergewebezelle, die durch ein die Leber betreffendes chemisches Karzinogen transformiert wurde, wobei die Polyprenylcarbonsäure 3,7,11,15-Tetramethyl-2,4,6,10,14-hexadecapentaensäure ist.

2. Eine Polyprenylverbindung zur Verwendung gemäß Anspruch 1, wobei die Polyprenylcarbonsäure (2E,4E,6E, 10E)-3,7,11,15-Tetramethyl-2,4,6,10,14-hexadecapentaensäure ist.

3. Eine Polyprenylverbindung zur Verwendung gemäß Anspruch 1 oder 2, wobei die Lebergewebezelle eine Ovale Zelle ist.

4. Eine Polyprenylverbindung zur Verwendung gemäß irgendeinem der Ansprüche 1 bis 3, die zur Hemmung der Transformation einer Zelle verwendet wird, die von Hepatitis- und/oder Zirrhose betroffen ist.

5. Eine Polyprenylverbindung zur Verwendung gemäß irgendeinem der Ansprüche 1 bis 4, in Form einer pharmazeutischen Zusammensetzung enthaltend einen pharmazeutisch akzeptablen Träger.

6. Eine Polyprenylverbindung zur Verwendung gemäß Anspruch 5, als pharmazeutische Zusammensetzung zur oralen Verabreichung.

## Revendications

1. Acide carboxylique polyprénylique en tant que principe actif pour une utilisation dans l'inhibition de l'installation, de la récurrence (oncogenèse secondaire), d'une modification maligne d'un hépatome par l'inhibition de l'expression du TGF-α dans une cellule de tissu hépatique transformée par un carcinogène chimique hépatique, où l'acide carboxylique polyprénylique est l'acide 3,7,11,15-tétraméthyl-2,4,6,10,14-hexadécapentaénoïque.

2. Composé polyprénylique pour une utilisation selon la revendication 1, où l'acide carboxylique polyprénylique est l'acide (2E,4E,6F,10E)-3,7,13,15-tétraméthyl-2,4,6,10,14-hexadécapentaénoïque.

3. Composé polyprénylique pour une utilisation selon la revendication 1 ou 2, où la cellule de tissu hépatique est une cellule ovale.

4. Composé polyprénylique pour une utilisation selon l'une quelconque des revendications 1 à 3, qui est utilisé pour l'inhibition de la transformation d'une cellule d'hépatite et/ou de cirrhose.

5. Composé polyprénylique pour une utilisation selon l'une quelconque des revendications 1 à 4, qui est sous la forme d'une composition pharmaceutique comprenant un support pharmaceutiquement acceptable.

6. Composé polyprénylique pour une utilisation selon la revendication 5, qui est une composition pharmaceutique pour une administration orale.
